# EUROPEAN PATENT SPECIFICATION

(11) **EP 3 201 194 B1**
(45) Date of publication and mention of the grant of the patent: **29.05.2019**
(21) Application number: 16788256.2
(22) Date of filing: 04.08.2016
(51) Int. Cl.: C07D 417/04, C07D 235/28, C07D 235/32, C07D 409/06, A61K 31/4184, A61P 1/04, A61P 35/00, A61P 33/00

(54) **SALTS OF COMPOUNDS HAVING A BENZIMIDAZOLIC STRUCTURE, USES AND PROCESS FOR THE PREPARATION THEREOF**
SALZE VON VERBINDUNGEN MIT BENZIMIDAZOLSTRUKTUR, VERWENDUNGEN UND VERFAHREN ZUR HERSTELLUNG DAVON
SELS DE COMPOSÉS AYANT UNE STRUCTURE BENZIMIDAZOLIQUE, LEURS UTILISATIONS ET PROCÉDÉ DE PRÉPARATION

(30) Priority: 06.08.2015 IT UB20152959
(43) Date of publication of application: 09.08.2017
(73) Proprietor: Istituto Superiore di Sanità, 00161 Roma (IT); Università Degli Studi, 66013 Chieti Scalo (CH) (IT); Università Degli Studi di Roma "La Sapienza", 00185 Roma (RM) (IT)
(72) Inventor: CIRILLI, Roberto, 00161 ROMA (RM) (IT); CARRADORI Simone, 66013 Chieti Scalo (CH) (IT); CASULLI Adriano, 00161 ROMA (RM) (IT); DE MONTE, Celeste, 00185 Roma (IT)
(74) Representative: Gitto, Serena
(86) International application number: PCT/IT2016/000191
(87) International publication number: WO 2017/021992

(56) References cited:
- DE-A1- 2 307 519
- GB-A- 2 025 223
- US-A- 3 538 108
- CHASSAING C ET AL: "Highly water-soluble prodrugs of anthelmintic benzimidazole carbamates: synthesis, pharmacodynamics, and pharmacokinetics", JOURNAL OF MEDICINAL CHEMISTRY, AMERICAN CHEMICAL SOCIETY, US, vol. 51, no. 5, 13 March 2008 (2008-03-13) , pages 1111-1114, XP009135085, ISSN: 0022-2623, DOI: 10.1021/JM701456R [retrieved on 2008-02-14]
- KAVITA RATHORE ET AL: "Synthesis and Antibiotic Activity of Mebendazole Derivatives of Pharmacological Interest", E-JOURNAL OF CHEMISTRY, vol. 4, no. 2, 1 January 2007 (2007-01-01) , pages 166-172, XP055241376, IN ISSN: 0973-4945, DOI: 10.1155/2007/503952

## Description

The present invention relates to salts of compounds having a benzimidazolic structure, uses thereof and a process for the preparation thereof.

Disclosed are salts of anthelmintic compounds with a benzimidazolic structure, namely, albendazole (ABZ), Fenbendazole (FBZ), Triclabendazole (TRBZ), or sulphoxides thereof, flubendazole (FLZ), mebendazole (MBZ), oxibendazole (OBZ), thiabendazole (TBZ), cambendazole (CBZ), parbendazole (PBZ) and nocodazole (NCZ), the use thereof and a process for the preparation thereof.

Anthelmintic compounds containing a benzimidazolic (BZD) nucleus represent an important class of drugs used worldwide in the treatment and prevention of parasitic diseases that develop in farms animals, pets and humans.

For example, neglected tropical diseases (NTDs) are a group of infectious diseases that mainly affect the poorer segments of modern societies, especially rural populations and the most disadvantaged urban ones. Unexpectedly, these diseases are present, and on the rise, also in emerging market economies including the group of the twenty most industrialised countries in the world (G20). More than a billion people in the world are affected by these diseases, which are defined as "neglected" because they are characterized by scant attention from political decision makers, a lack of priority within health care strategies, inadequate research and the allocation of limited resources. Among NTDs, helminthiases transmitted from the ground (caused mainly by *Ascaris lumbricoides*, *Trichuris trichiura*, *Necator americanus* and *Ancylostoma duodenale*) are responsible for the largest health impact. Recent estimates indicate that in 2010, more than 1.4 billion people were infected with at least one of these 4 species of helminths, with a global health impact of around 5.2 million DALYs (disability-adjusted life years).

In recent years, the World Health Organization (WHO) has promoted the concept of integrated preventive chemotherapy as the main strategy for controlling and eliminating many helminthiases. For example, in 2013 alone, the WHO administered 200 million doses of albendazole (ABZ) and mebendazole (MBZ).

Due to their interaction with microtubules, in recent years some anthelmintic drugs have been extensively studied also as antitumour agents. The published experimental results are indicative of a significant clinical potential for the use of ABZ, ABZ sulphoxide (ABZ-SO), MBZ and nocodazole (NCZ) in the treatment of several tumour forms. In particular, MBZ has demonstrated cytotoxicity against cell lines of glioblastoma multiforme (the most common and most aggressive brain cancer) and ovarian carcinoma (the most lethal malignant gynecological tumour).

However, due to their low water solubility, these drugs cannot be administered parenterally and if administered orally do not reach the systemic circulation in concentrations making them suitable for use in antitumour therapies. Therefore, for a careful evaluation on a systemic scale of the antitumour activity of β-tubuiin inhibiting anthelmintic drugs it is necessary to use new pharmaceutical formulations that are more water soluble.

At present, anthelmintic drugs are used in veterinary and human therapy only in a non-salified form, with a sole exception that will be described here below, and due to their limited water solubility they are formulated only for oral use in tablets or suspensions. Following oral administration, their absorption is limited and variable and only a small percentage of the drug reaches the systemic circulation. Therefore, in order to produce the desired pharmacological action it is necessary to use a very high dose of the drug.

It has been attempted to remedy the poor solubility of anthelmintic drugs by using organic co-solvents and surfactants or by complexing with β-cydodextrin and derivatives (e.g. HP-β-CD), but the solubility achieved has not been sufficient to prepare suitable injectable products.

Albendazole sulphoxide (ABZ-SO), also called ricobendazole (RBZ), is the only anthelmintic used in the veterinary practice in a salified form, more specifically in the form of a hydrochloride. RBZ is poorly soluble in water and slightly soluble in the majority of injectable co-solvents or surfactants. The products Bayverm Pi® (Bayer Argentina) and Sintyotal-R® (Biogenesis Argentina), highly acidic solutions in which RBZ is present in a concentration of between 10 and 15% (w/v), are presently marketed as injectable. The adjustment of the pH to very high levels of acidity enables the protonation of the basic nitrogen of the imidazole ring and the consequent formation of a water-soluble salt. The bioavailability of these products after subcutaneous injection in cattle does not reach over 40%. This low level of bioavailability has been attributed to post-injection precipitation of the active ingredient and a slow and incomplete redissolution at the injection site. Moreover, the post-injection precipitation of the drug is responsible for possible negative effects such as mechanical irritation or thrombosis in the tissues at the injection site.

The sulphinyl-benzimidazolic anthelmintic drugs ABZ, fenbendazole (FBZ) and triclabendazole (TRBZ) are rapidly biotransformed *in vivo* into the corresponding sulphoxides (ABZ-SO, FBZ-SO and TRBZ-SO, respectively), which are then further oxidated into sulphones. In vivo, the carbamate group of the sulphone is hydrolyzed to amino sulphone as a consequence of the hydrolytic splitting of the amide bond. Both the sulphur and sulphoxide forms show anthelmintic activity, whilst the sulphone and amino sulphone are inactive metabolites.

The ABZ-SO, FBZ-SO and TRBZ-SO forms have a stereogenic centre consisting of the sulphur atom of the sulphoxide group. In vivo studies have demonstrated that the plasma concentration of the dextrorotatory form of ABZ-SO ((+)-ABZ-SO) is predominant over the levorotatory form ((-)-ABZ-SO) in patients treated with ABZ. Moreover, an accumulation of (+)-ABZ-SO has been observed in the cerebrospinal fluid of patients with neurocysticercosis.

The pharmacokinetic studies available for ABZ-SO have demonstrated that the relative plasma concentrations of the dextro- and levorotatory enantiomers of ABZ-SO vary according to species, age and gender of the host animal and species of parasite. The (-)-ABZ-SO enantiomer has shown to be predominant in rats and mice, whereas the (+)-ABZ-SO enantiomer is the prevalent species in sheep, goats, dogs, cattle and humans. These data point to a stereoselectivity in drug/organism interaction and suggest a potential therapeutic application of the individual enantiomers.

In order to verify the biological activity and efficacy of enantiomers of sulphoxides and evaluate their efficacy in future clinical studies it is however mandatory to obtain more soluble forms of the individual enantiomers in a simple and economical manner.

However, the ABZ-SO, FBZ-SO and TRBZ-SO sulphoxides are only slightly soluble in ethanol and methanol and cannot be resolved in discrete amounts, for example by HPLC resolution. In particular, the solubility of ABZ-SO (ricobendazole) in ethanol is 1.36 mg/ml whereas in water it is 0.062 mg/ml (JOURNAL OF PHARMACEUTICAL SCIENCES, VOL. 94, NO. 5, MAY 2005). Moreover, no asymmetric synthetic processes capable of producing the two enantiomers of ABZ-SO, FBZ-SO and TRBZ-SO with a high degree of optical purity are known in the literature.

In light of the foregoing, therefore, the need to be able to have new forms of anthelmintic compounds with a benzimidazolic structure that overcome the advantages of the known forms is clearly apparent.

Disclosed herein are drugs albendazole (ABZ), fenbendazole (FBZ), triclabendazole (TRBZ), flubendazole (FLZ), mebendazole (MBZ), oxibendazole (OBZ), thiabendazole (TBZ), cambendazole (CBZ), parbendazole (PBZ) and nocodazole (NCZ) which are more water soluble than the starting compounds, easily obtainable synthetically and in high yields.

An object of the present invention is to provide new more soluble forms of chiral compounds of albendazole sulphoxide (ABZ-SO), fenbendazole sulphoxide (FBZ-SO) and triclabendazole sulphoxide (TRBZ-SO), possibly usable as individual enantiomers, which can be obtained using processes that are cost-effective and easy to implement on a preparative, semi-industrial or industrial scale.

According to the present invention, therefore, new salts of the above-mentioned compounds were prepared, said salts being more water soluble than the non-salified compounds. This chemical transformation enables a parenteral, as well as oral, administration of the drug, greater bioavailability and the attainment of optimal therapeutic levels at lower doses than those envisaged for the non-salified forms.

Unlike sulphinyl-benzimidazolic compounds, which are not chiral, the ABZ-SO, FBZ-SO and TRBZ-SO sulphoxides according to the invention are chiral in that the presence of a sulphur stereogenic centre of the sulphoxide group imparts chirality to the system. It is therefore possible, by varying the stereochemistry of the sulphoxides, to modulate the biological response and minimize side effects, an extremely advantageous strategy for compounds intended for human use.

It is known that the poor solubility of a racemic sample in the mobile phase (< 1 mg mL⁻¹) is a limiting factor in the processes of enantiomer separation on an industrial scale. For example, *ABZ-SO* is practically insoluble in n-hexane and water and slightly soluble in ethanol (circa 1 mg mL⁻¹) and methanol.

Unlike non-salified sulphoxides, the salts of the ABZ-SO, FBZ-SO and TRBZ-SO sulphoxides according to the present invention exhibit good solubility in ethanol and methanol (>10 mg/ml), which permits the resolution of the raceme by HPLC. For example, the *ABZ-SO-Na* salt according to the present invention has a solubility in ethanol and in methanol of about 30 mg mL⁻¹.

Therefore, according to the present invention, the enantiomers with an absolute (R) and (S) configuration of the salts of the ABZ-SO, FBZ-SO and TRBZ-SO sulphoxides were obtained by HPLC resolution of the racemic salts using Pirkle-type stationary chiral phases or ones based on polysaccharide derivatives. The mobile phases used in the enantiomeric separation consist of mixtures of hexane/alcohol (methanol, ethanol or 2-propanol), hexane/organic modifier (dichloromethane, chloroform, THF, dioxane, ethyl acetate), hexane/alcohol/organic modifier (alcohol and modifier as described above), pure organic solvents such as methanol, ethanol, ethyl acetate or acetonitrile or hydro-organic mixtures containing methanol, ethanol or acetonitrile and water, wherein the percentage of water ranges from 1 to 60% (v/v). In particular, the HPLC resolution was conducted on an amount of racemic compound comprised between 5 and 20 mg per chromatography run using chiral columns based on polysaccharide derivatives (Chiralpak IA, Chiralpak IB, Chiralpak IC, Chiralpak ID, Chiralpak IE, Chiralpak IF, Chiralpak AD, Chiralpak AS) with a geometry of 250 mm x 10 mm I.D. and mobile phases consisting of pure methanol or ethanol. The enantiomers isolated by enantioselective HPLC showed enantiomeric excesses greater than 98%.

Preliminary water solubility tests showed that the salts according to the present invention are more soluble than the corresponding non-salified compounds, the effect of salification on solubility showing to be more marked for the sulphoxides.

The synthetic strategy for preparing the salts of the invention and the salts herein disclosed comprises a single simple salification step, wherein the nitrogen atom of the imidazole nucleus with acidic characteristics is deprotonated by a base capable of releasing a cation A⁺ⁿ = Li⁺, Na⁺, K⁺, Mg⁺² or Ca⁺². Unlike the non-salified forms, the saline forms with A⁺ⁿ = Li⁺, Na⁺, K⁺ are water soluble.

The structures of the salts of the benzimidazolic anthelmintic compounds, possibly chiral, are shown below: 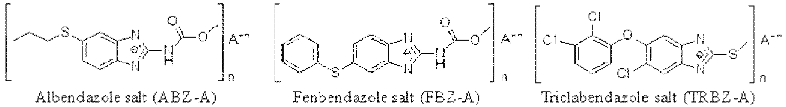 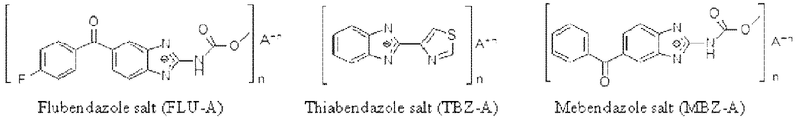 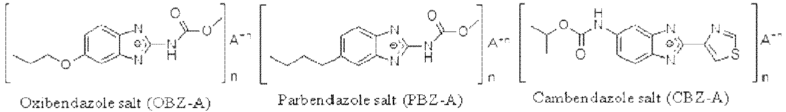 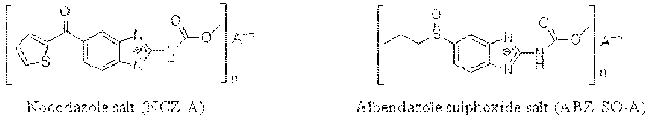 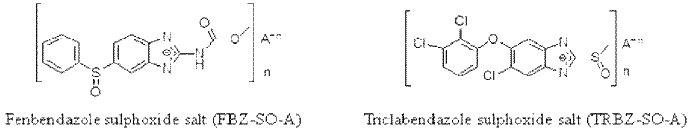 wherein:

| | |
|---|---|
| n | A⁺ⁿ |
| 1 | Li⁺ |
| | Na⁺ |
| | K⁺ |
| 2 | Mg⁺² |
| | Ca⁺² |

Therefore, the specific subject matter of the present invention relates to salts of benzimidazolic compounds with metals selected in the group consisting of lithium, sodium, potassium, magnesium or calcium, when said benzimidazolic compounds are selected in the group consisting of albendazole sulphoxide (ABZ-SO), and triclabendazole sulphoxide (TRBZ-SO), or with metals selected in the group consisting of lithium, potassium, magnesium and calcium, when the benzimidazolic compound is fenbendazole sulphoxide, said sulphoxides being in racemic form or in the form of an R- or S-enantiometer.

According to a preferred embodiment of the present invention, the metals are Na⁺ or K⁺.

Moreover, the salts according to the present invention can be complexed with β-cyclodextrin or derivatives thereof with the aim of further increasing the solubility of the compounds.

The present invention further relates to a pharmaceutical composition comprising or consisting of at least one salt, as the active ingredient, together with one or more excipients and/or adjuvants, wherein said at least one salt is a salt of benzimidazolic compounds with metals selected in the group consisting of lithium, sodium, potassium, magnesium and calcium, said benzimidazolic compounds being selected in the group consisting of albendazole sulphoxide, fenbendazole sulphoxide and triclabendazole sulphoxide, said sulphoxides being in racemic form or in the form of the R- or S-enantiomer.

The pharmaceutical composition according to the present invention can further comprise at least one drug selected in the group consisting in anthelmintics, antitumourals and proton pump inhibitors.

Among the anthelmintic drugs, the following can be used: abamectin, praziquantel, diethylcarbamazine, niclosamide, ivermectin, suramin, pirantel, levamisole, octadepsipeptides such as, for example, emodepside, aminoacetonitrile derivatives such as, for example, monepantel, and spiroindoles such as, for example, derquantel.

Among the antitumour drugs, mention can be made, for example, of taxanes such as, for example, paclitaxel, docetaxel and cabazitaxel, camptothecins such as, for example, irinotecan and topotecan, Vinca alkaloids, platinum complexes such as, for example, oxaliplatin, cisplatin and carboplatin, temozolomide, gemcitabin, capecitabine and bevacizumab.

The proton pump inhibitors that can be used according to the present invention are for example omeprazole, lansoprazole, rabeprazole, and pantoprazole, in a racemic or enantiomerically pure form, or salts thereof.

The present invention further relates to a salt of benzimidazolic compounds with metals selected in the group consisting of lithium, sodium, potassium, magnesium and calcium, said benzimidazolic compounds being selected in the group consisting of albendazole sulphoxide, fenbendazole sulphoxide and triclabendazole sulphoxide, said sulphoxides being in racemic form or in the form of the R- or S-enantiomer or the pharmaceutical composition as defined above for use as a medication.

In particular, the salt or the pharmaceutical composition as defined above can be advantageously used as anthelmintics or antitumourals according to claims 8-9.

The tumours that can be treated according to the present invention are, for example, ovarian carcinoma, adrenocortical carcinoma, lung carcinoma, pancreatic tumours, breast tumours, colorectal cancer, kidney tumours, melanoma, glioblastoma multiforme, osteosarcoma, leukaemia and lymphomas.

The present invention further relates to the combination of at least one salt with at least one further anthelmintic drug, for separate or sequential use in the treatment of helminthiases, wherein said at least one salt is a salt of benzimidazolic compounds with metals selected in the group consisting of lithium, sodium, potassium, magnesium and calcium, said benzimidazolic compounds being selected in the group consisting of albendazole sulphoxide, fenbendazole sulphoxide and triclabendazole sulphoxide, said sulphoxides being in racemic form or in the form of the R- or S- enantiomer. Said at least one further anthelmintic drug can be selected in the group consisting in abamectin, praziquantel, diethylcarbamazine, niclosamide, ivermectin, suramin, pirantel, levamisole, octadepsipeptides such as emodepside, aminoacetonitrile derivatives such as monepantel, and spiroindoles such as derquantel.

The subject matter of the present invention also relates to the combination of at least one salt with at least one antitumour drug and/or proton pump inhibitor, for separate or sequential use in the treatment of tumours, wherein said at least one salt is a salt of benzimidazolic compounds with metals selected in the group consisting of lithium, sodium, potassium, magnesium and calcium, said benzimidazolic compounds being selected in the group consisting of albendazole sulphoxide, fenbendazole sulphoxide and triclabendazole sulphoxide, said sulphoxides being in racemic form or in the form of the R- or S-enantiomer.

Among antitumour drugs, use can be made of taxanes, such as paclitaxel, docetaxel and cabazitaxel, camptothecins such as irinotecan and topotecan, Vinca alkaloids, platinum complexes such as oxaliplatin, cisplatin and carboplatin, temozolomide, gemcitabine, capecitabine and bevacizumab.

The tumours that can be treated according to the present invention are, for example, ovarian carcinoma, adrenocortical carcinoma, lung carcinoma, pancreatic tumours, breast tumours, colorectal cancer, kidney tumours, melanoma, glioblastoma multiforme, osteosarcoma, leukaemia and lymphoma.

Among proton pump inhibitors, use can be made of omeprazole, lansoprazole, pantoprazole and rabeprazole, in a racemic or enantiomerically pure form, or salts thereof.

According to the present invention, "separate use" is meant to indicate the simultaneous administration of the two or more compounds of the combination according to the invention in pharmaceutically distinct forms.

The term "sequential use" is meant to indicate the successive administration of the two or more compounds of the composition according to the invention, each in a pharmaceutically distinct form.

The subject matter of the present invention further relates to a process for the preparation of the salts as defined in claim 1 said process comprising or consisting in the following steps: a) mixing a benzimidazolic compound selected in the group consisting in albendazole sulphoxide (ABZ-SO), fenbendazole sulphoxide (FBZ-SO) and triclabendazole sulphoxide (TRBZ-SO), said sulphoxides being in racemic form or in the form of an R- or S-enantiomer, with a base capable of releasing a cation selected in the group consisting in lithium, sodium, potassium, magnesium or calcium in a suitable solvent, such as, for example, ethanol, methanol, 2-propanol or 1-propanol, pure or in hydroalcoholic mixtures; b) allowing to react until the formation of the salt. Step b) of the process according to the present invention can be conducted at a temperature ranging from room temperature to the reflux temperature.

The reaction mixture is cooled and filtered and the solvent is made to evaporate, for example under reduced pressure.

The salts disclosed herein are generally obtainable from active ingredients of low-cost generic drugs via a single synthetic step which runs with quantitative yields. For example, ABZ, the starting product for the synthesis of the salt ABZ-A, presently costs about one thousand euro per kg.

Examples of bases capable of releasing the cation are LiOH, NaOH, KOH or LiOR, LiNH2, LiNR2, NaOR, NaNH2, NaNR2, KOR, KNH2 or KNR2, where R is an ethyl group.

As stated above, a solvent suitable for use in the process of the present invention is for example ethanol; other suitable solvents are methanol, 2-propanol and propanol, pure or in hydroalcoholic mixtures. Therefore, salts with A⁺ = Li⁺, Na⁺ or K⁺ are prepared treating the neutral form of the benzimidazolic compounds listed above with LiOH, NaOH or KOH in ethanol or with LiOR, LiNH2, LiNR2, NaOR, NaNH2, NaNR2, KOR, KNH2 or KNR2, where R is an ethyl group.

The salts with Aⁿ⁺ = Na⁺ or K⁺ can be converted into another salt with n=2 and Aⁿ⁺ = Mg⁺² or Ca⁺² with an identical formula via an exchange of cations. When the starting salt and final salt product are sufficiently soluble in the reaction environment, this exchange can be achieved using a cationic exchange resin saturated with the cation desired in the product. The exchange can also be carried out by exploiting the lower solubility of the desired salt. Based on this principle, for example, the ion Na⁺ can be exchanged with Ca²⁺ or Mg²⁺.

Moreover, when the salts of the invention are the salts of albendazole sulphoxide, fenbendazole sulphoxide or triclabendazole sulphoxide, the process of the invention can further comprise a step c) of separating the enantiopure forms of said salts, for example by HPLC on polysaccharide-based chiral stationary phases.

In addition the present invention concernes a process for the preparation of salts of fenbendazole sulphoxide with sodium, said sulphoxide being in racemic form or in the form of the R- or S-enantiomer, said process comprising or consisting of the following steps: a) mixing a fenbendazole sulphoxide, said sulphoxide being in racemic form or in the form of an R- or S-enantiomer, with a base capable of releasing a cation that is sodium; b) allowing to react until the formation of the salt; and c) separating the enantiopure forms of said salts by HPLC on polysaccharide-based chiral stationary phases, according to claims 18-20, 22.

The invention will be described below by way of illustration and not by way of limitation, with particular reference to some illustrative examples.

### EXAMPLE 1: Preparation of the salts of benzimidazolic compounds according to the present invention

### Reference example

***ABZ-Na synthesis**.* A weighed amount of sodium hydroxide (1.0 eq) was solubilised in 40 mL of ethanol. ABZ (1.0 eq) was then added to the ethanol solution. The reaction mixture was heated under reflux for 2 hours. At the end of the reaction the mixture was cooled and filtered. The solvent was evaporated under reduced pressure. ABZ sodium salt appeared as a pale pink-coloured solid; mp 269-270 °C; IR vₘₐₓ 3338 (v N-H), 1708 (v C=O), 1626 (v C=N), 1583 (v C=C), 1262 (v C-N), 1085 (v S=O), 733 (δ Cₛₚ₂-H) cm⁻¹; ¹H NMR (400 MHz, DMSO-*d₆*): *δ* 0.94-1.01 (m, 3H, CH₃), 1.52-1.57 (m, 2H, CH₂), 2.78-2.82 (m, 2H, CH₂), 3.69 (s, 3H, OCH₃), 6.95-6.97 (m, 1H, Ar), 7.27 (d, *J* = 8.0 Hz, 1H, Ar), 7.45 (s, 1H, Ar), 12.80 (bs, 1H, NH, D₂O exch.); ¹³C NMR (101 MHz, DMSO-*d*₆) *δ* 13.55 (*C*H₃), 22.67 (*C*H₃*C*H₂), 38.12 (*C*H₂S), 51.56 (O*C*H₃), 112.98 (Ar), 117.24 (Ar), 123.20 (Ar), 123.60 (Ar), 139.00 (Ar), 140.45 (Ar), 158.97 (*C*=O), 160.36 (*C*=N).

A synthetic procedure identical to the one shown for obtaining ABZ-Na was applied for the other salts of the present invention.

### Reference example

***ABZ-K.*** White-coloured solid, yield = 98%, mp 270-271 °C; IR v ₘₐₓ 3239 (v N-H), 1608 (v C=N), 1561 (v C=C), 1270 (v C-N), 787 (δ Cₛₚ₂-H) cm⁻¹; ¹H NMR (400 MHz, DMSO-*d₆*): *δ* 0.95 (t, *J* = 7.2 Hz, 3H, CH₃), 1.50-1.55 (m, 2H, CH₂), 2.77-2.80 (m, 2H, CH₂), 3.57 (s, 3H, OCH₃), 6.91 (d, *J* = 8.0 Hz, 1H, Ar), 7.13 (d, *J* = 8.0 Hz, 1H, Ar), 7.28 (s, 1H, Ar), 12.01 (bs, 1H, NH, D₂O exch.); ¹³C NMR (101 MHz, DMSO-*d*₆) *δ* 13.54 (*C*H₃), 22.64 (*C*H₃*C*H₂), 38.03 (*C*H₂S), 51.64 (O*C*H₃), 112.76 (Ar), 115.93 (Ar), 123.19 (Ar), 123.60 (Ar), 139.12 (Ar), 141.15 (Ar), 158.93 (*C*=O), 161.36 (*C*=N).

***ABZ-SO-Na.*** White-coloured solid, yield = 97%, mp 271-274 °C; IR v ₘₐₓ 3188 (v N-H), 1704 (v C=O), 1618 (v C=N), 1581 (v C=C), 1264 (v C-N), 1194 (v C-O), 1098 (v S=O), 734 (δ Cₛₚ₂-H) cm⁻¹; ¹H NMR (400 MHz, DMSO-*d₆*): *δ* 3.64 (s, 3H, OCH₃), 7.06-7.12 (m, 4H, Ar), 7.25 (t, *J* = 7.6 Hz, 2H, Ar), 7.40 (d, *J* = 8.0 Hz, 1H, Ar), 7.58 (s, 1H, Ar), 12.93 (bs, 1H, NH, D₂O exch.); ¹³C NMR (101 MHz, DMSO-*d*₆) *δ* 51.78 (O*C*H₃), 114.10 (Ar), 120.00 (Ar), 125.57 (Ar), 126.01 (Ar), 127.07 (Ar), 129.41 (Ar), 140.52 (Ar), 157.55 (*C*=O), 158.86 (*C*=N).

***ABZ-SO-K.*** White-coloured solid, yield = 97%, mp 262-264 °C; IR v ₘₐₓ 3228 (v N-H), 1704 (v C=O), 1610 (v C=N), 1560 (v C=C), 1263 (v C-N), 1185 (v C-O), 1097 (v S=O), 734 (δ Cₛₚ₂-H) cm⁻¹; ¹H NMR (400 MHz, DMSO-*d₆*): *δ* 3.63 (s, 3H, OCH₃), 7.09-7.14 (m, 4H, Ar), 7.25 (t, *J* = 7.6 Hz, 2H, Ar), 7.39 (d, *J* = 8.0 Hz, 1H, Ar), 7.52 (s, 1H, Ar), 12.84 (bs, 1H, NH, D₂O exch.); ¹³C NMR (101 MHz, DMSO-*d*₆) *δ* 51.84 (O*C*H₃), 114.13 (Ar), 119.22 (Ar), 120.71 (Ar), 123.61 (Ar), 125.72 (Ar), 126.29 (Ar), 127.29 (Ar), 129.46 (Ar), 139.64 (Ar), 140.18 (Ar), 156.40 (*C*=O), 158.90 (*C*=N).

### Reference example

***FBZ-Na.*** White-coloured solid, yield = 96%, mp 270-272 °C; IR v ₘₐₓ 3362 (v N-H), 1726 (v C=O), 1624 (v C=N), 1507 (v C=C), 1271 (v C-N), 1192 (v C-O), 1093 (v S=O), 748 (δ Cₛₚ₂-H) cm⁻¹; ¹H NMR (400 MHz, DMSO-*d₆*): *δ* 3.71 (s, 3H, OCH₃), 7.28 (d, *J* = 8.0 Hz, 1H, Ar), 7.42-7.53 (m, 4H, Ar), 7.63-7.68 (m, 3H, Ar); ¹³C NMR (101 MHz, CD₃OD) *δ* 51.21 (O*C*H₃), 110.75 (Ar), 113.20 (Ar), 117.39 (Ar), 124.31 (Ar), 128.93 (Ar), 130.50 (Ar), 133.30 (Ar), 140.01 (Ar), 143.18 (Ar), 145.09 (Ar), 159.74 (*C*=O), 160.72 (*C*=N).

### Reference example

***FBZ-K.*** White-coloured solid, yield = 98%, mp 238-240 °C; 3336 (v N-H), 1712 (v C=O), 1621 (v C=N), 1522 (v C=C), 1269 (v C-N), 1189 (v C-O), 1083 (v S=O), 746 (δ Cₛₚ₂-H) cm⁻¹; ¹H NMR (400 MHz, DMSO-*d₆*): *δ* 3.56 (s, 3H, OCH₃), 7.11-7.14 (m, 1H, Ar), 7.25 (d, *J* = 8.0 Hz, 1H, Ar), 7.44-7.52 (m, 4H, Ar), 7.61 (d, *J* = 7.2 Hz, 2H, Ar), 11.83 (bs, 1H, NH, D₂O exch.); ¹³C NMR (101 MHz, DMSO-*d*₆) *δ* 51.66 (O*C*H₃), 109.34 (Ar), 112.86 (Ar), 116.21 (Ar), 124.43 (Ar), 129.54 (Ar), 130.62 (Ar), 134.26 (Ar), 139.69 (Ar), 143.11 (Ar), 147.76 (Ar), 160.58 (*C*=O), 160.89 (*C*=N).

### Reference example

***TRBZ-Na.*** Yellow-coloured solid, yield = 96%, mp >250 °C (dec.); IR vₘₐₓ 3112 (v Cₛₚ₂-H), 1613 (v C=N), 1573 (v C=C), 1271 (v C-N), 733 (δ Cₛₚ₂-H) cm⁻¹; ¹H NMR (400 MHz, DMSO-*d₆*): *δ* 2.91 (s, 3H, SCH₃), 6.95 (s, 1H, Ar), 7.13-7.39 (m, 3H, Ar), 8.02 (s, 1H, Ar); ¹³C NMR (101 MHz, DMSO-*d*₆) *δ* 35.60 (S*C*H₃), 106.32 (Ar), 116.33 (Ar), 118.21 (Ar), 120.56 (Ar), 122.02 (Ar), 126.77 (Ar), 135.11 (Ar), 137.41 (Ar), 137.98 (Ar), 141.00 (Ar), 154.01 (OAr), 154.09 (OAr), 160.95 (*C*=N).

### Reference example

***TRBZ-K.*** Yellow-coloured solid, yield = 98%, mp >250 °C (dec.); IR vₘₐₓ 3106 (v Cₛₚ₂-H), 1604 (v C=N), 1571 (v C=C), 1279 (v C-N), 755 (δ Cₛₚ₂-H) cm⁻¹; ¹H NMR (400 MHz, DMSO-*d₆*): *δ* 2.89 (s, 3H, SCH₃), 6.90 (s, 1H, Ar), 7.09-7.40 (m, 3H, Ar), 7.97 (s, 1H, Ar); ¹³C NMR (101 MHz, DMSO-*d*₆) *δ* 35.55 (S*C*H₃), 105.14 (Ar), 115.55 (Ar), 118.68 (Ar), 120.33 (Ar), 123.32 (Ar), 126.56 (Ar), 135.69 (Ar), 136.11 (Ar), 137.34 (Ar), 140.76 (Ar), 153.45 (OAr), 153.67 (OAr), 161.11 (*C*=N).

***TRBZ-SO-Na.*** Brown-coloured solid, yield 96%, mp >250 °C (dec.); IR v ₘₐₓ 3105 (v Cₛₚ₂-H), 1634 (v C=N), 1590 (v C=C), 1248 (v C-N), 1077 (v S=O), 723 (δ Cₛₚ₂-H) cm⁻¹; ¹H NMR (400 MHz, DMSO-*d₆*): *δ* 3.11 (s, 3H, SOCH₃), 7.12 (s, 1H, Ar), 7.29-7.45 (m, 3H, Ar), 8.27 (s, 1H, Ar); ¹³C NMR (101 MHz, DMSO-*d*₆) *δ* 54.86 (S*C*H₃), 108.87 (Ar), 117.57 (Ar), 119.24 (Ar), 121.65 (Ar), 124.34 (Ar), 126.89 (Ar), 133.45 (Ar), 134.76 (Ar), 136.76 (Ar), 141.56 (Ar), 152.07 (OAr), 153.55 (OAr), 159.67 (*C*=N).

***TRBZ-SO-K.*** Brown-coloured solid, yield = 99%, mp >250 °C (dec.); IR v ₘₐₓ 3110 (v Cₛₚ₂-H), 1628 (v C=N), 1577 (v C=C), 1286 (v C-N), 1088 (v S=O), 727 (δ Cₛₚ₂-H) cm⁻¹; ¹H NMR (400 MHz, DMSO-*d₆*): *δ* 3.15 (s, 3H, SOCH₃), 7.03 (s, 1H, Ar), 7.22-7.56 (m, 3H, Ar), 8.04 (s, 1H, Ar); ¹³C NMR (101 MHz, DMSO-*d*₆) *δ* 54.04 (S*C*H₃), 107.98 (Ar), 116.43 (Ar), 118.12 (Ar), 120.54 (Ar), 124.55 (Ar), 127.13 (Ar), 136.08 (Ar), 136.87 (Ar), 137.52 (Ar), 141.33 (Ar), 153.12 (OAr), 153.79 (OAr), 160.76 (*C*=N).

### Reference example

***Thiabendazole-Na (TBZ-A).*** White-coloured solid, yield = 96%, mp 299-300 °C; IR vₘₐₓ 3085 (v Cₛₚ₂-H), 2985 (v Cₛₚ₃-H), 1405 (v C=C), 1229 (v C-N), 740 (δ Cₛₚ₂-H) cm⁻¹; ¹H NMR (300 MHz, DMSO-*d₆*): *δ* 7.17-7.20 (q, 2H, Ar), 7.54-7.57 (q, 2H, Ar), 8.42-8.43 (d, *J* = 2.4 Hz, 1H, Ar), 9.31-9.32 (d, *J* = 1.8 Hz, 1H, Ar); ¹³C NMR (75 MHz, DMSO-*d*₆) *δ* 119.85 (Ar), 122.58 (Ar), 147.50 (Ar), 147.57 (Ar), 156.06 (*C*=N).

### Reference example

***Mebenbendazole-Na (MBZ-A).*** Yellow-coloured solid, yield = 94%, mp 288-289 °C (dec.); IR v ₘₐₓ 3199 (v N-H), 1619 (v C=N), 1563 (v C=C), 1280 (v C-N), 791 (δ Cₛₚ₂-H) cm⁻¹; ¹H NMR (300 MHz, DMSO-*d₆*): δ 3.44 (s, 3H, CH₃), 6.97-6.99 (d, *J* = 8.1 Hz, 1H, Ar), 7.10-7.13 (dd, 1H, Ar), 7.42-7.60 (m, 6H, Ar); ¹³C NMR (75 MHz, DMSO-*d*₆) δ 51.18 (OCH₃), 111.16 (Ar), 115.37 (Ar), 120.28 (Ar), 123.21 (Ar), 128.17 (Ar), 129.27 (Ar), 130.31 (Ar), 141.82 (*C*=N), 161.17 (NHCO), 195.60 (CO).

### Reference example

***Oxibendazole-Na (OBZ-A).*** Pink-coloured solid, yield = 97%, mp 249-251 °C (dec.); IR v ₘₐₓ 3388 (v N-H), 2963 (v Cₛₚ₃-H), 1626 (v C=N), 1268 (v C-N), 1179 (v C-O), 792 (δ Cₛₚ₂-H) cm⁻¹; ¹H NMR (300 MHz, DMSO-*d₆*): *δ* 0.93-0.98 (t, *J* = 7.3 Hz, 3H, CH₃), 1.65-1.72 (m, 2H, CH₂), 3.63 (s, 3H, CH₃), 3.82-3.87 (t, *J* = 6.4 Hz, 2H, CH₂), 6.54-6.57 (dd, 1H, Ar), 6.85-6.86 (d, *J* = 2.4 Hz, 1H, Ar), 7.13-7.15 (d, *J* = 8.1 Hz, 1H, Ar); ¹³C NMR (75 MHz, DMSO-*d*₆) *δ* 10.97 (CH₃), 22.67 (CH₂), 52.39 (OCH₃), 69.84 (OCH₂), 109.39 (Ar), 114.69 (Ar), 154.32 (*C*=N).

### Reference example

***Parbendazole-Na (PBZ-A).*** Pink-coloured solid, yield = 95%, mp 247-248 °C (dec.); IR v ₘₐₓ 3327 (v N-H), 2963 (v Cₛₚ₃-H), 1625 (v C=N), 1594 (v C=C), 1280 (v C-N), 1195 (v C-O), 790 (δ Cₛₚ₂-H) cm⁻¹; ¹H NMR (300 MHz, DMSO-*d₆*): *δ* 0.84-0.89 (t, *J* = 7.3 Hz, 3H, CH₃), 1.26-1.31 (m, 2H, CH₂), 1.48-1.55 (m, 2H, CH₂), 3.64 (s, 3H, CH₃), 6.76-6.79 (dd, 1H, Ar), 7.10-7.18 (m, 2H, Ar); ¹³C NMR (75 MHz, DMSO-*d*₆) *δ* 14.30 (CH₃), 22.18 (CH₂), 35.58 (CH₂), 39.07 (CH₂), 52.39 (OCH₃), 113.09 (Ar), 113.14 (Ar), 121.27 (Ar), 121.38 (Ar), 134.85 (Ar), 134.91 (Ar), 150.70 (*C*=N), 157.27 (NHCO).

### EXAMPLE 2: Preparation of the enantiopure forms of the salt ABZ-SO-Na.

Enantiomeric separation of *ABZ-SO-Na* by semi-preparative HPLC was conducted on 1-cm I.D. Chiralpak AD or Chiralpak IA columns, progressively increasing the amount of racemic mixture injected in a single phase. The mobile phase consisted of pure ethanol. The flow temperature and flow velocity were fixed at 25°C and 4.5 ml/min.

The injection of 15 mg of *ABZ-SO-Na* dissolved in 1 mL of methanol resulted in a complete enantioseparation, without peak overlapping, in a run time of about 15 min. After the semipreparative separation, the collected fractions were pooled, subjected to evaporation and analyzed by means of a chiral analytical column in order to determine their enantiomeric excess (e.e.). Both collected fractions were enantiomerically pure and the quantitative yields were about 90%. Therefore, the productivity of the sample of enantiomers of *ABZ-SO-Na* was about 650 mg per day.

## Claims

1. Salts of benzimidazolic compounds with metals selected in the group consisting of lithium, sodium, potassium, magnesium and calcium, when said benzimidazolic compounds are selected in the group consisting of albendazole sulphoxide and triclabendazole sulphoxide, or with metals selected in the group consisting of lithium, potassium, magnesium and calcium, when the benzimidazolic compound is fenbendazole sulphoxide, said sulphoxides being in racemic form or in the form of the R- or S-enantiomer.

2. Pharmaceutical composition comprising or consisting of at least one salt, as an active ingredient, together with one or more excipients and/or adjuvants, wherein said at least one salt is a salt of benzimidazolic compounds with metals selected in the group consisting of lithium, sodium, potassium, magnesium and calcium, said benzimidazolic compounds being selected in the group consisting of albendazole sulphoxide, fenbendazole sulphoxide and triclabendazole sulphoxide, said sulphoxides being in racemic form or in the form of the R- or S-enantiomer.

3. Pharmaceutical composition according to claim 2, further comprising at least one drug selected of the group consisting in anthelmintics, antitumourals and proton pump inhibitors.

4. Pharmaceutical composition according to claim 3, wherein said at least one anthelmintic drug is selected in the group consisting of abamectin, praziquantel, diethylcarbamazine, niclosamide, ivermectin, suramin, pirantel, levamisole, octadepsipeptides such as emodepside, aminoacetonitrile derivatives such as monepantel, and spiroindoles such as derquantel.

5. Pharmaceutical composition according to claim 3, wherein said at least one antitumour drug is selected in the group consisting of taxanes such as paclitaxel, docetaxel and cabazitaxel, camptothecins such as irinotecan and topotecan, Vinca alkaloids, platinum complexes such as oxaliplatin, cisplatin and carboplatin, temozolomide, gemcitabine, capecitabine and bevacizumab.

6. Pharmaceutical composition according to claim 3, wherein said at least one proton pump inhibitor drug is selected in the group consisting of omeprazole, lansoprazole, rabeprazole, and pantoprazole, in a racemic or enantiomerically pure form, or salts thereof.

7. Salt of benzimidazolic compounds with metals selected in the group consisting of lithium, sodium, potassium, magnesium and calcium, said benzimidazolic compounds being selected in the group consisting of albendazole sulphoxide, fenbendazole sulphoxide and triclabendazole sulphoxide, said sulphoxides being in racemic form or in the form of the R- or S-enantiomer or pharmaceutical composition as defined in any one of claims 2-6, for use as a medication.

8. Salt of benzimidazolic compounds with metals selected in the group consisting of lithium, sodium, potassium, magnesium and calcium, said benzimidazolic compounds being selected in the group consisting of albendazole sulphoxide, fenbendazole sulphoxide and triclabendazole sulphoxide, said sulphoxides being in racemic form or in the form of the R- or S-enantiomer or pharmaceutical composition as defined in any one of claims 2-6, for use in the treatment of helminthiases.

9. Salt of benzimidazolic compounds with metals selected in the group consisting of lithium, sodium, potassium, magnesium and calcium, said benzimidazolic compounds being selected in the group consisting of albendazole sulphoxide, fenbendazole sulphoxide and triclabendazole sulphoxide, said sulphoxides being in racemic form or in the form of the R- or S-enantiomer or pharmaceutical composition as defined in any one of claims 2-6, for use in the treatment of tumours.

10. Salt as defined in claim 9 or pharmaceutical composition as defined in any one of claims 2-6, for use according to claim 9, wherein said tumour is selected in the group consisting of ovarian carcinoma, adrenocortical carcinoma, lung carcinoma, pancreatic tumours, breast tumours, colorectal cancer, kidney tumours, melanoma, glioblastoma multiforme, osteosarcoma, leukaemia and lymphomas.

11. Combination of at least one salt with at least one drug anthelmintic, for separate or sequential use in the treatment of helminthiases, wherein said at least one salt is a salt of benzimidazolic compounds with metals selected in the group consisting of lithium, sodium, potassium, magnesium and calcium, said benzimidazolic compounds being selected in the group consisting of albendazole sulphoxide, fenbendazole sulphoxide and triclabendazole sulphoxide, said sulphoxides being in racemic form or in the form of the R- or S-enantiomer.

12. Combination according to claim 11, for use according to claim 11, wherein said at least one further anthelmintic drug is selected in the group consisting of abamectin, praziquantel, diethylcarbamazine, niclosamide, ivermectin, suramin, pirantel, levamisole, octadepsipeptides such as emodepside, aminoacetonitrile derivatives such as monepantel, and spiroindoles such as derquantel.

13. Combination of at least one salt with at least one antitumour drug and/or proton pump inhibitor, for separate or sequential use in the treatment of tumours, wherein said at least one salt is a salt of benzimidazolic compounds with metals selected in the group consisting of lithium, sodium, potassium, magnesium and calcium, said benzimidazolic compounds being selected in the group consisting of albendazole sulphoxide, fenbendazole sulphoxide and triclabendazole sulphoxide, said sulphoxides being in racemic form or in the form of the R- or S-enantiomer.

14. Combination according to claim 13, for use according to claim 13, wherein said at least one further antitumour drug is selected in the group consisting of taxanes such as paclitaxel, docetaxel and cabazitaxel, camptothecins such as, for example, irinotecan and topotecan, Vinca alkaloids, platinum complexes such as oxaliplatin, cisplatin and carboplatin, temozolomide, gemcitabin, capecitabine and bevacizumab.

15. Combination according to any one of claims 13-14, for use according to claim 13, wherein said tumour is selected in the group consisting of ovarian carcinoma, adrenocortical carcinoma, lung carcinoma, pancreatic tumours, breast tumours, colorectal cancer, kidney tumours, melanoma, glioblastoma multiforme, osteosarcoma, leukaemia and lymphomas.

16. Combination according to claim 13, for use according to claim 13, wherein said at least one proton pump inhibitor is selected in the group consisting of omeprazole, lansoprazole, rabeprazole, and pantoprazole, in a racemic or enantiomerically pure form, or salts thereof.

17. Process for the preparation of the salts as defined in claim 1, said process comprising or consisting of the following steps: a) mixing a benzimidazolic compound selected in the group consisting in albendazole sulphoxide, fenbendazole sulphoxide or triclabendazole sulphoxide, said sulphoxides being in racemic form or in the form of an R- or S-enantiomer, with a base capable of releasing a cation selected in the group consisting in lithium, sodium, potassium, magnesium or calcium in a suitable solvent; b) allowing to react until the formation of the salt.

18. Process for the preparation of salts of fenbendazole sulphoxide with sodium, said sulphoxide being in racemic form or in the form of the R- or S-enantiometer, said process comprising or consisting of the following steps: a) mixing a fenbendazole sulphoxide, said sulphoxide being in racemic form or in the form of an R- or S-enantiometer, with a base capable of releasing a cation that is sodium; b) allowing to react until the formation of the salt; and c) separating the enantiopure forms by HPLC on polysaccharide-based chiral stationary phases.

19. Process according to anyone of claims 17-18, wherein said suitable solvent is selected in the group consisting in ethanol, methanol, 2-propanol, 1-propanol, pure or in hydroalcoholic mixtures.

20. Process according to any one of claims 17-19, wherein step b) is conducted at a temperature ranging from room temperature to the reflux temperature.

21. Process according to any one of claims 17, 19-20, wherein the base capable of releasing the cation is selected in the group consisting of LiOH, NaOH, KOH or LiOR, LiNH2, LiNR2, NaOR, NaNH2, NaNR2, KOR, KNH2 or KNR2, where R is an ethyl group.

22. Process according to any one of claims 18-20, wherein the base capable of releasing the cation is selected in the group consisting of NaOH, NaOR, NaNH2, NaNR2, wherein R is an ethyl group.

23. Process according to any one of claims 17, 19-22, wherein said process further comprises a step c) of separating the enantiopure forms of said salts.

24. Process according to claim 23, wherein the separation of the enantiopure forms of said salts is conducted by HPLC on polysaccharide-based chiral stationary phases.

## Patentansprüche

1. Salze von benzimidazolischen Verbindungen mit Metallen ausgewählt in der Gruppe bestehend aus Lithium, Natrium, Kalium, Magnesium und Calcium, wenn die benzimidazolischen Verbindungen in der Gruppe ausgewählt werden bestehend aus Albendazolsulfoxid und Triclabendazolsulfoxid, oder mit Metallen ausgewählt in der Gruppe bestehend aus Lithium, Kalium, Magnesium und Calcium, wenn die benzimidazolische Verbindung Fenbendazolsulfoxid ist, wobei die Sulfoxide in razemischer Form oder in Form des R- oder S-Enantiomers vorliegen.

2. Pharmazeutische Zusammensetzung umfassend mindestens ein oder bestehend aus mindestens einem Salz als Wirkstoff zusammen mit einem oder mehr Trägerstoffen und/oder Hilfsmitteln, wobei das mindestens eine Salz ein Salz von benzimidazolischen Verbindungen mit Metallsalzen ist ausgewählt in der Gruppe bestehend Lithium, Natrium, Kalium, Magnesium und Calcium, wobei die benzimidazolischen Verbindungen in der Gruppe ausgewählt werden bestehend aus Albendazolsulfoxid, Fenbendazolsulfoxid und Triclabendazolsulfoxid, wobei die Sulfoxide in razemischer Form oder in Form des R- oder S-Enantiomers vorliegen

3. Pharmazeutische Zusammensetzung nach Anspruch 2, ferner mindestens ein Medikament umfassend ausgewählt in der Gruppe bestehend aus Anthelmintika, Antitumormitteln und Protonenpumpenhemmern.

4. Pharmazeutische Zusammensetzung nach Anspruch 3, wobei das mindestens eine anthelmintische Medikament in der Gruppe ausgewählt wird bestehend aus Abamectin, Praziquantel, Diethylcarbamazin, Niclosamid, I vermectin, Suramin, Pirantel, Levamisol, Octadepsipeptiden wie Emodepsid, Aminoacetonitrilderivaten wie Monepantel und Spiroindolen wie Derquantel.

5. Pharmazeutische Zusammensetzung nach Anspruch 3, wobei das mindestens eine Anti-Tumormedikament in der Gruppe ausgewählt wird bestehend aus Taxanen wie Paclitaxel, Docetaxel und Cabazitaxel, Camptothecinen wie Irinotecan und Topotecan, Vincaalkaloiden, Platinkomplexen wie Oxaliplatin, Cisplatin und Carboplatin, Temozolomid, Gemcitabin, Capecitabin und Bevacizumab.

6. Pharmazeutische Zusammensetzung nach Anspruch 3, wobei das mindestens eine Protonenpumpenhemmermedikament in der Gruppe ausgewählt wird bestehend aus Omeprazol, Lansoprazol, Rabeprazol und Pantoprazol in einer razemischen oder enantiomer reinen Form oder Salzen davon.

7. Salz benzimidazolischer Verbindungen mit Metallen ausgewählt in der Gruppe bestehend aus Lithium, Natrium, Kalium, Magnesium und Calcium, wobei die benzimidazolischen Verbindungen in der Gruppe ausgewählt werden bestehend aus Albendazolsulfoxid, Fenbendazolsulfoxid und Triclabendazolsulfoxid, wobei die Sulfoxide in razemicher Form oder in Form des R- oder S-Enantiomers vorliegen, oder pharmazeutische Zusammensetzung wie in irgendeinem der Ansprüche 2-6 definiert, zur Verwendung als Medikament.

8. Salz benzimidazolischer Verbindungen mit Metallen ausgewählt in der Gruppe bestehend aus Lithium, Natrium, Kalium, Magnesium und Calcium, wobei die benzimidazolischen Verbindungen in der Gruppe ausgewählt werden bestehend aus Albendazolsulfoxid, Fenbendazolsulfoxid und Triclabendazolsulfoxid, wobei die Sulfoxide in razemischer Form oder in Form des R- oder S-Enantiomers vorliegen, oder pharmazeutische Zusammensetzung wie in irgendeinem der Ansprüche 2-6 definiert, zur Verwendung bei der Behandlung von Helminthiasen.

9. Salz benzimidazolischer Verbindungen mit Metallen ausgewählt in der Gruppe bestehend aus Lithium, Natrium, Kalium, Magnesium und Calcium, wobei die benzimidazolischen Verbindungen in der Gruppe ausgewählt werden bestehend aus Albendazolsulfoxid, Fenbendazolsulfoxid und Triclabendazolsulfoxid, wobei die Sulfoxide in razemischer Form oder in Form des R- oder S-Enantiomers vorliegen, oder pharmazeutische Zusammensetzung wie in irgendeinem der Ansprüche 2-6 definiert, zur Verwendung bei der Behandlung von Tumoren.

10. Salz wie in Anspruch 9 definiert oder pharmazeutische Zusammensetzung wie in irgendeinem der Ansprüche 2-6 definiert zur Verwendung nach Anspruch 9, wobei der Tumor in der Gruppe ausgewählt wird bestehend aus Eierstockkarzinom, Nebennierenrindenkarzinom, Lungenkarzinom, Bauchspeicheldrüsentumoren, Brusttumoren, kolorektalem Krebs, Nierentumoren, Melanomen, Gliobastoma multiforme, Osteosarkomen, Leukämie und Lymphomen.

11. Kombination von mindestens einem Salz mit mindestens einem Arzneimittelantehlminthikum zur getrennten oder sequenzielle Verwendung bei der Behandlung von Helminthiasen, wobei das mindestens eine Salz ein Salz benzimidazolischer Verbindungen mit Metallen ausgewählt in der Gruppe bestehend aus Lithium, Natrium, Kalium, Magnesium und Calcium ist, wobei die benzimidazolischen Verbindungen in der Gruppe ausgewählt werden bestehend aus Albendazolsulfoxid, Fenbendazolsulfoxid und Triclabendazolsulfoxid, wobei die Sulfoxide in razemischer Form oder in Form des R- oder S-Enantiomers vorliegen.

12. Kombination nach Anspruch 11 zur Verwendung nach Anspruch 11, wobei mindestens ein weiteres anthelminthisches Medikament in der Gruppe ausgewählt wird bestehend aus Abamectin, Praziquantel, Diethylcarbamazin, Niclosamid, Ivermectin, Suramin, Pirantel, Levamisol, Octadepsipeptiden wie Emodepsid, Aminoacetonitrilderivaten wie Monepantel und Spiroindolen wie Derquantel.

13. Kombination von mindestens einem Salz mit mindestens einem Anti-Tumormedikament und/oder Protonenpumpenhemmer zur getrennten oder sequenzielle Verwendung bei der Behandlung von Tumoren, wobei das mindestens eine Salz ein Salz benzimidazolischer Verbindungen mit Metallen ausgewählt in der Gruppe bestehend aus Lithium, Natrium, Kalium, Magnesium und Calcium ist, wobei die benzimidazolischen Verbindungen in der Gruppe ausgewählt werden bestehend aus Albendazolsulfoxid, Fenbendazolsulfoxid und Triclabendazolsulfoxid, wobei die Sulfoxide in razemischer Form oder in Form des R- oder S-Enantiomers vorliegen.

14. Kombination nach Anspruch 13 zur Verwendung nach Anspruch 13, wobei das mindestens eine weitere Anti- Tumormedikament in der Gruppe ausgewählt wird bestehend aus Taxanen wie Paclitaxel, Docetaxel und Cabazitaxel, Camptothecinen wie beispielsweise Irinotecan und Topotecan, Vincaalkaloiden, Platinkomplexen wie Oxaliplatin, Cisplatin und Carboplatin, Temozolomid, Gemcitabin, Capecitabin und Bevacizumab.

15. Kombination nach irgendeinem der Ansprüche 13-14 zur Verwendung nach Anspruch 13, wobei der Tumor in der Gruppe ausgewählt wird bestehend aus Eierstockkarzinom, Nebennierenrindenkarzinom, Lungenkarzinom, Bauchspeicheldrüsentumoren, Brusttumoren, kolorektalem Krebs, Nierentumoren, Melanomen, Gliobastoma multiforme, Osteosarkomen, Leukämie und Lymphomen.

16. Kombination nach Anspruch 13 zur Verwendung nach Anspruch 13, wobei mindestens ein Protonenpumpenhemmer in der Gruppe ausgewählt wird bestehend aus Omeprazol, Lansoprazol, Rabeprazol und Pantoprazol in einer razemischen oder enantiomer reinen Form, oder Salze davon.

17. Verfahren für die Herstellung der Salze wie in Anspruch 1 definiert, wobei das Verfahren die folgenden Schritte umfasst oder daraus besteht: a) Mischen einer benzimidazolischen Verbindung ausgewählt in der Gruppe bestehend aus Albendazolsulfoxid, Fenbendazolsulfoxid oder Triclabendazolsulfoxide, wobei die Sulfoxide in razemischer Form oder in Form des R- oder S-Enantiomers vorliegen, mit einer Base, die in der Lage ist, ein Kation freizusetzen, das in der Gruppe ausgewählt wird bestehend aus Lithium, Natrium, Kalium, Magnesium oder Calcium, in einem geeigneten Lösungsmittel;
b) Gestatten des Reagierens bis zur Bildung des Salzes.

18. Verfahren für die Herstellung von Salzen von Fenbendazolsulfoxid mit Natrium, wobei das Sulfoxid in razemischer oder in Form des R- oder S-Enantiomers vorliegt, wobei das Verfahren die folgenden Schritte umfasst oder daraus besteht: a) Mischen eines Fenbendazolsulfoxids , wobei das Sulfoxid in razemischer Form oder in Form eines R- oder S-Enantiomers vorliegt, mit einer Base, die in der Lage ist, ein Kation freizusetzen, das Natrium ist; b) Gestatten des Reagierens bis zur Bildung des Salzes; und c) Trennen der enantiomerenreinen Formen durch HPLC an chiralen stationären Phasen auf der Basis von Polysaccharid.

19. Verfahren nach irgendeinem der Ansprüche 17-18, wobei das geeignete Lösungsmittel in der Gruppe ausgewählt wird bestehend aus Ethanol, Methanol, 2-Propranol, 1-Propranol, reinen Mischungen oder in hydroalkoholischen Mischungen.

20. Verfahren nach irgendeinem der Ansprüche 17-19, wobei der Schritt b) bei einer Temperatur im Bereich von Raumtemperatur bis zur Rückflusstemperatur ausgeführt wird.

21. Verfahren nach irgendeinem der Ansprüche 17, 19-20, wobei die Base, die in der Lage ist, das Kation freizusetzen, in der Gruppe ausgewählt wird bestehend aus LiOH, NaOH, KOH oder LiOR, LiNH2, LiNR2, NaOR, NaNH2, NaNR2, KOR, KNH2 oder KNR2, wobei R eine Ethylgruppe ist.

22. Verfahren nach irgendeinem der Ansprüche 18-20, wobei die Base, die in der Lage ist, das Kation freizusetzen, in der Gruppe ausgewählt wird bestehend aus NaOH, NaOR, NaNH2, NaNR2, wobei R eine Ethylgruppe ist.

23. Verfahren nach irgendeinem der Ansprüche 17, 19-22, wobei das Verfahren ferner einen Schritt c) des Abtrennens der enantiomerenreinen Formen der Salze umfasst.

24. Verfahren nach Anspruch 23, wobei die Trennung der enantiomerenreinen Formen der Salze durch HPLC an chiralen stationären Phasen auf der Basis von Polysaccharid ausgeführt wird.

## Revendications

1. Sels de composés benzimidazoliques avec des métaux sélectionnés parmi le groupe constitué du lithium, du sodium, du potassium, du magnésium et du calcium, lorsque lesdits composés benzimidazoliques sont sélectionnés parmi le groupe constitué du sulphoxyde d'albendazole et du sulphoxyde de triclabendazole, ou avec des métaux sélectionnés parmi le groupe constitué du lithium, du potassium, du magnésium et du calcium, lorsque le composé benzimidazolique est du sulphoxyde de fenbendazole, lesdits sulphoxydes étant sous forme racémique ou sous forme de l'énantiomère R ou S.

2. Composition pharmaceutique comprenant ou étant constituée d'au moins un sel, en tant qu'ingrédient actif, associé à un ou plusieurs excipients et/ou adjuvants, dans laquelle ledit au moins un sel est un sel de composés benzimidazoliques avec des métaux sélectionnés parmi le groupe constitué du lithium, du sodium, potassium, du magnésium et du calcium, lesdits composés benzimidazoliques étant sélectionnés parmi le groupe constitué du sulphoxyde d'albendazole, du sulphoxyde de fenbendazole et du sulphoxyde de triclabendazole, lesdits sulphoxydes étant sous forme racémique ou sous forme de l'énantiomère R ou S.

3. Composition pharmaceutique selon la revendication 2, comprenant en outre au moins un médicament sélectionné parmi le groupe constitué des anthelminthiques, des antitumoraux et des inhibiteurs de la pompe à protons.

4. Composition pharmaceutique selon la revendication 3, dans laquelle ledit au moins un médicament anthelminthique est sélectionné parmi le groupe constitué de l'abamectine, du praziquantel, de la diéthylcarbamazine, de la niclosamide, de l'ivermectine, de la suramine, du pyrantel, du lévamisole, des octadepsipeptides tels que l'emodepside, des dérivés d'aminoacétonitrile tels que le monépantel, et des spiroindoles tels que le derquantel.

5. Composition pharmaceutique selon la revendication 3, dans laquelle ledit au moins un médicament antitumoral est sélectionné parmi le groupe constitué des taxanes tels que le paclitaxel, le docétaxel et le cabazitaxel, des camptothécines telles que l'irinotécan et le topotécan, des alcaloïdes de la pervenche, des complexes du platine tels que l'oxaliplatine, le cisplatine et le carboplatine, du témozolomide, de la gemcitabine, de la capécitabine et du bévacizumab.

6. Composition pharmaceutique selon la revendication 3, dans laquelle ledit au moins un médicament inhibiteur de pompe à protons est sélectionné parmi le groupe constitué de l'oméprazole, du lansoprazole, du rabéprazole, et du pantoprazole, sous forme racémique ou énantiomériquement pure, ou des sels de ceux-ci.

7. Sel de composés benzimidazoliques avec des métaux sélectionnés parmi le groupe constitué du lithium, du sodium, du potassium, du magnésium et du calcium, lesdits composés benzimidazoliques étant sélectionnés parmi le groupe constitué du sulphoxyde d'albendazole, du sulphoxyde de fenbendazole et du sulphoxyde de triclabendazole, lesdits sulphoxydes étant sous forme racémique ou sous forme de l'énantiomère R ou S ou une composition pharmaceutique selon l'une quelconque des revendications 2 à 6, destinée à être utilisée en tant que médicament.

8. Sel de composés benzimidazoliques avec des métaux sélectionnés parmi le groupe constitué du lithium, du sodium, du potassium, du magnésium et du calcium, lesdits composés benzimidazoliques étant sélectionnés parmi le groupe constitué du sulphoxyde d'albendazole, du sulphoxyde de fenbendazole et du sulphoxyde de triclabendazole, lesdits sulphoxydes étant sous forme racémique ou sous forme de l'énantiomère R ou S ou une composition pharmaceutique selon l'une quelconque des revendications 2 à 6, destinée à être utilisée dans le traitement de l'helminthose.

9. Sel de composés benzimidazoliques avec des métaux sélectionnés parmi le groupe constitué du lithium, du sodium, du potassium, du magnésium et du calcium, lesdits composés benzimidazoliques étant sélectionnés parmi le groupe constitué du sulphoxyde d'albendazole, du sulphoxyde de fenbendazole et du sulphoxyde de triclabendazole, lesdits sulphoxydes étant sous forme racémique ou sous forme de l'énantiomère R ou S ou une composition pharmaceutique selon l'une quelconque des revendications 2 à 6, destinée à être utilisée dans le traitement des tumeurs.

10. Sel selon la revendication 9 ou composition pharmaceutique selon l'une quelconque des revendications 2 à 6, destinée à être utilisée selon la revendication 9, dans lequel ladite tumeur est sélectionnée parmi le groupe constitué d'un carcinome ovarien, d'un carcinome corticosurrénal, d'un carcinome du poumon, de tumeurs pancréatiques, de tumeurs mammaires, d'un cancer colorectal, de tumeurs rénales, d'un mélanome, d'un glioblastome multiforme, d'un ostéosarcome, d'une leucémie et de lymphomes.

11. Combinaison d'au moins un sel avec au moins un médicament anthelminthique, destinée à être utilisée séparément ou séquentiellement dans le traitement de l'helminthose, dans laquelle ledit au moins un sel est un sel de composés benzimidazoliques avec des métaux sélectionnés parmi le groupe constitué du lithium, du sodium, du potassium, du magnésium et du calcium, lesdits composés benzimidazoliques étant sélectionnés parmi le groupe constitué du sulphoxyde d'albendazole, du sulphoxyde de fenbendazole et du sulphoxyde de triclabendazole, lesdits sulphoxydes étant sous forme racémique ou sous forme de l'énantiomère R ou S.

12. Combinaison selon la revendication 11, destinée à être utilisée selon la revendication 11, dans laquelle ledit au moins un médicament anthelminthique supplémentaire est sélectionné parmi le groupe constitué de l'abamectine, du praziquantel, de la diéthylcarbamazine, de la niclosamide, de l'ivermectine, de la suramine, du pyrantel, du lévamisole, des octadepsipeptides tels que l'emodepside, des dérivés d'aminoacétonitrile tels que le monépantel, et des spiroindoles tels que le derquantel.

13. Combinaison d'au moins un sel avec au moins un médicament antitumoral et/ou inhibiteur de pompe à protons, destinée à être utilisée séparément ou séquentiellement dans le traitement des tumeurs, dans laquelle ledit au moins un sel est un sel de composés benzimidazoliques avec des métaux sélectionnés parmi le groupe constitué du lithium, du sodium, du potassium, du magnésium et du calcium, lesdits composés benzimidazoliques étant sélectionnés parmi le groupe constitué du sulphoxyde d'albendazole, du sulphoxyde de fenbendazole et du sulphoxyde de triclabendazole, lesdits sulphoxydes étant sous forme racémique ou sous forme de l'énantiomère R ou S.

14. Combinaison selon la revendication 13, destinée à être utilisée selon la revendication 13, dans laquelle ledit au moins un médicament antitumoral supplémentaire est sélectionné parmi le groupe constitué des taxanes tels que le paclitaxel, le docétaxel et le cabazitaxel, des camptothécines telles que, par exemple, l'irinotécan et le topotécan, des alcaloïdes de la pervenche, des complexes du platine tels que l'oxaliplatine, le cisplatine et le carboplatine, du témozolomide, de la gemcitabine, de la capécitabine et du bévacizumab.

15. Combinaison selon l'une quelconque des revendications 13 à 14, destinée à être utilisée selon la revendication 13, dans laquelle ladite tumeur est sélectionnée parmi le groupe constitué d'un carcinome ovarien, d'un carcinome corticosurrénal, d'un carcinome du poumon, de tumeurs pancréatiques, de tumeurs mammaires, d'un cancer colorectal, de tumeurs rénales, d'un mélanome, d'un glioblastome multiforme, d'un ostéosarcome, d'une leucémie et de lymphomes.

16. Combinaison selon la revendication 13, destinée à être utilisée selon la revendication 13, dans laquelle ledit au moins un inhibiteur de pompe à protons est sélectionné parmi le groupe constitué de l'oméprazole, du lansoprazole, du rabéprazole, et du pantoprazole, sous forme racémique ou énantiomériquement pure, ou des sels de ceux-ci.

17. Procédé pour la préparation de sels selon la revendication 1, ledit procédé comprenant ou étant constitué des étapes suivantes consistant à : a) mélanger un composé benzimidazolique sélectionné parmi le groupe constitué du sulphoxyde d'albendazole, du sulphoxyde de fenbendazole ou du sulphoxyde de triclabendazole, lesdits sulphoxydes étant sous forme racémique ou sous forme de l'énantiomère R ou S, avec une base apte à libérer un cation sélectionné parmi le groupe constitué du lithium, du sodium, du potassium, du magnésium ou du calcium dans un solvant approprié; b) laisser réagir jusqu'à la formation du sel.

18. Procédé pour la préparation de sels de sulphoxyde de fenbendazole avec du sodium, ledit sulphoxyde étant sous forme racémique ou sous forme de l'énantiomère R ou S, ledit procédé comprenant ou étant constitué des étapes suivantes consistant à : a) mélanger du sulphoxyde de fenbendazole, ledit sulphoxyde étant sous forme racémique ou sous forme de l'énantiomère R ou S, avec une base apte à libérer un cation qui est du sodium ; b) laisser réagir jusqu'à la formation du sel ; et c) séparer des formes énantiopures par CLHP sur des phases stationnaires chirales à base de polysaccharides.

19. Procédé selon l'une quelconque des revendications 17 à 18, dans lequel ledit solvant approprié est sélectionné parmi le groupe constitué de l'éthanol, du méthanol, du 2-propanol, du 1-propanol, pur ou dans des mélanges hydroalcooliques.

20. Procédé selon l'une quelconque des revendications 17 à 19, dans lequel l'étape b) est mise en oeuvre à une température allant de la température ambiante à la température de reflux.

21. Procédé selon l'une quelconque des revendications 17, 19 à 20, dans lequel la base apte à libérer le cation est sélectionnée parmi le groupe constitué de LiOH, NaOH, KOH ou LiOR, LiNH2, LiNR2, NaOR, NaNH2, NaNR2, KOR, KNH2 ou KNR2, où R est un roupe éthyle.

22. Procédé selon l'une quelconque des revendications 18 à 20, dans lequel la base apte à libérer le cation est sélectionnée parmi le groupe constitué de NaOH, NaOR, NaNH2, NaNR2, dans lequel R est un groupe éthyle.

23. Procédé selon l'une quelconque des revendications 17, 19 à 22, dans lequel ledit procédé comprend en outre une étape c) de séparation des formes énantiopures desdits sels.

24. Procédé selon la revendication 23, dans lequel la séparation des formes énantiopures desdits sels est mise en oeuvre par CLHP sur des phases stationnaires chirales à base de polysaccharides.
